# EUROPEAN PATENT SPECIFICATION

(11) **EP 4 025 584 B1**
(45) Date of publication and mention of the grant of the patent: **25.02.2026**
(21) Application number: 20764430.3
(22) Date of filing: 04.09.2020
(51) Int. Cl.: C07K 1/14, C12N 1/08, C12N 7/02

(54) **COMPOSITIONS AND METHODS FOR REDUCING CHROMATIN CONTENT OF BIOLOGICAL PREPARATIONS**
ZUSAMMENSETZUNGEN UND VERFAHREN ZUR VERRINGERUNG DES CHROMATINGEHALTS VON BIOLOGISCHEN PRÄPARATEN
COMPOSITIONS ET PROCÉDÉS POUR RÉDUIRE LA TENEUR EN CHROMATINE DE PRÉPARATIONS BIOLOGIQUES

(30) Priority: 06.09.2019 EP 19195973; 11.02.2020 EP 20156662
(43) Date of publication of application: 13.07.2022
(73) Proprietor: Sartorius BIA Separations d.o.o., 5270 Ajdovscina (SI)
(72) Inventor: GAGNON, Peter Stanley, NV 89101 (US); MAKOVSEK, Vesna, 2317 Oplotnica (SI)
(74) Representative: Novagraaf International SA
(86) International application number: PCT/EP2020/074861
(87) International publication number: WO 2021/044029

(56) References cited:
- WO-A1-2013/180647
- WO-A1-2015/183180
- US-A1- 2017 130 208

## Description

### Background of The Invention

The amount of DNA in a final drug formulation is a primary safety and regulatory concern for all biological drugs. This includes for example adeno-associated viruses (AAV) that are employed as delivery vehicles for therapeutic DNA plasmids in the field of gene therapy. The safety concern is not the therapeutic DNA inside the AAV capsids but rather the DNA left from the host cells used to produce the AAV. Removing this host-derived DNA has been recognized as an obstacle to the progress of the field because it has proven more difficult to remove than anticipated. The possibility exists that the difficulty may derive partly from host-derived DNA being bound to the external surface of the capsids.

The therapeutic DNA payload inside the AAV capsids is commonly referred to as vector DNA. Some of this DNA may also be present outside the capsids, either due to incomplete insertion or later damage to initially filled capsids. This DNA is not considered to be a primary safety concern, but it is an issue because of its potential ability to inflate the apparent numbers of DNA-filled capsids as measured by various DNA assays. Sample preparation methods are available to limit the influence of extra-capsid vector DNA, but it would be preferable if it was simply absent.

Treatment of AAV harvests with nuclease enzymes to lyse host-derived DNA is a common feature of AAV purification procedures. A limitation of this approach is that host DNA is protected against enzymatic lysis by its strong association with histone proteins. Substantial amounts of host DNA are not digested and they remain in the AAV preparation. Some purification procedures additionally employ anion exchange chromatography to increase overall reduction of host DNA. The rationale is that DNA should bind to the anion exchanger more strongly than AAV, making it possible to elute the AAV while DNA remains on the anion exchanger. Such treatment does reduce host DNA content but does not eliminate it completely, and its failure to do so relates to the same issue that limits the effectiveness of enzymatic treatment: Host cell DNA in harvests exists in strong associations with histone proteins. In addition to physically shielding the DNA from enzymatic lysis, the strongly associated histones create hybrid aggregations with intermediate charge characteristics so that a subset of the DNA still co-elutes with the virus [1].

Alternatively, a subset of host DNA still associated with histones can be precipitated from cell culture harvests by reducing the pH of harvest. This method was described by in 1994, using citrate buffers at physiological salt concentrations to titrate pH into the range of pH 2.6 to pH 5.0 [2]. This work was conducted on mammalian cell culture harvests but the technique has been since applied to bacteria, yeasts, fungus and insect cells [3-5]. It has been recently applied to mammalian cell culture harvests in the same range of pH and salt concentration for flocculation of AAV contaminants, including host DNA [6].

Harvest clarification and reduction of host cell DNA contamination has also been described using cationic heterocyclic compounds and soluble cationic polymers [7-10]. Anionic fatty acids and heterocyclic anions are also known to reduce DNA levels [11-16]. A common feature of these approaches is that they employ salt concentrations of less than 0.5 M (500 mM), such as in the range of about 15-300 mM salt, and most often in the range of 50-100 mM salt. Allantoin has been used alone and in combination with many of these agents and conditions [1,15,16]. It is known to have a high affinity for endotoxins [17].

It is known that DNA has pKₐ of about 2.6. It is known that host DNA in cell culture harvests is initially associated with histone proteins in nucleosomal arrays of various sizes. Histone proteins are extremely hydrophobic and have isoelectric points ranging from about 9 to 11. Host DNA partially dissociates from histone proteins in 1 M NaCl and dissociates increasingly as NaCl concentration is raised to 4 M, but it is not fully dissociated even at that concentration. Histone proteins begin to form insoluble aggregates at NaCl concentrations greater than 1 M. The dissociated DNA is soluble under high salt conditions but re-associates with histone proteins if the salt concentration is diminished.

Surfaces coated with primary amine ligands are known to bind biomolecules more strongly than surfaces with quaternary amine ligands. This includes virus particles, DNA, endotoxins, and acidic proteins. They generally bind these molecules at modestly higher salt concentrations than quaternary amines on similar surfaces. DNA also elutes from primary amine ligands at modestly higher salt concentrations but still less than 1 M sodium chloride. Experiments with quaternary amine surfaces and with primary amine surfaces are most often conducted at neutral to mildly alkaline pH (pH 7.0 to 8.5). Treatment of mammalian cell culture harvests with hydrophobic quaternary amine (cholestyramine) particles at neutral pH and salt concentrations of 50 to 150 mM sodium chloride is known to bind some DNA in mammalian cell culture harvests [18]. Subsequent removal of the particles results in a corresponding reduction of DNA content in the harvest.

[19] discloses a method of purifying a sample containing a desired protein including the steps of (i) providing a packed chromatographic column having positively charged porous particles, (ii) equilibrating the column to the conditions to which the desired protein in the sample is to elute, (iii) contacting the sample with the packed chromatographic column such that the sample volume applied to the packed chromatographic column is less than or equal to the interparticle space of the positively charged porous particles within the packed chromatographic column, (iv) eluting the desired protein from the packed chromatographic column, where the desired protein is in a purer state and in the conditions to which the packed chromatographic column was equilibrated; where the desired protein is an antibody, an antibody fragment, an antibody derivative, or an antibody fusion protein. In essence, [19] is about removing virus and yielding a desired protein whereas virus is unwanted. This reference is silent with respect to conserving a virus while removing DNA.

[20] discloses methods for enhancing reduction of both virus and viral DNA levels in protein preparations by contacting protein preparations with a surface bearing a primary amine, secondary amine, or both. However, this reference is silent with respect to remove DNA from virus and leaving the virus purified from viral DNA.

### References

[1] R. Nian, P. Gagnon, Advance chromatin extraction enhances performance and productivity of cation exchange chromatography-based capture of Immunoglobulin G monoclonal antibodies, J. Chromatogr. A 1453 (2016) 54-61.
[2] B. Lydersen, T. Brehm-Gibson, A. Murel, Acid precipitation of mammalian cell fermentation broth, Ann. N. Y. Acad. Sci. 745 (1994) 222-231.
[3] M. Westoby, J. Chrostowski, P. de Vilmorin, J.P. Smelko, J.K. Romero, Effects of solution environment on mammalian cell fermentation broth properties: enhanced impurity removal and clarification performance, Biotechnol. Bioeng. 108 (2011) 50-58.
[4] E. Soares, Quantification of yeast flocculation, J. Inst. Brew. 103 (1997) 93-98.
[5] J. Westman, M. Taherzadeh, C. Franzen, Inhibitor tolerance and flocculation of a yeast strain suitable for second generation bioethanol production, Electronic J. Biotechnol. 15 (2012) DOI: 10.2225/vol15-issue3-fulltext-8
[6] US 20170130208 A1.
[7] D. Salt, O. Hay, O. Thomas, M. Hoare, P. Dunhill, Selective flocculation of cellular contaminants from soluble proteins using polyethyleneimine: a study of several organisms and polymer molecular weights. Enz. Microbiol. Technol. 17 (1995) 107-113.
[8] J. Hughes, D. Ramsden, K. Symes, The flocculation of bacteria using cationic synthetic flocculants and chitosan. Biotechnol Tech. 4 (1990) 55-60.
[9] EP 0240 348.
[10] T. McNerney, A. Thomas, A.Senczuk, K. Petty, X. Zhao, R. Piper, J. Carvalho, M. Hammond, S. Sawant, and J. Bussiere, PDADMAC flocculation of Chinese hamster ovary cells: Enabling a centrifuge-less harvest process for monoclonal antibodies. MAbs 7 (2015) 413-427.
[11] A. Chantuin, R. Curnish, The precipitation of plasma proteins by short-chain fatty acids Arch. Biochem. Biophys. 89 (1960) 218-220.
[12] M. McInney, A. Parkinson, A simple, nonchromatographic procedure to purify immunoglobulins from serum and ascites fluid, J. Immunol. Met., 96 (1987) 271-278.
[13] US 8,063,189 B2.
[14] Y. Brodsky, C. Zhang, Y. Yigsaw, G. Vedanthum, Caprylic acid precipitation for impurity reduction: an alternative to conventional chromatography for monoclonal antibody purification, Biotechnol. Bioeng. 109 (2012) 2589-2598.
[15] P. Gagnon, R. Nian, Y-S. Yang, Q-Y. Yang, C-L. Lim, Non-immunospecific association of immunoglobulin G with chromatin during elution from protein A inflates host contaminants, aggregate content, and antibody loss, J. Chromatogr. A 1408 (2015) 151-160.
[16] R. Nian, W. Zhang, L. Tan, J. Lee, X. Bi, Y-S. Yang, H-T. Gan, P. Gagnon, Advance chromatin extraction improves capture performance of protein A affinity chromatography, J. Chromatogr. A 1431 (2016) 1-7.
[17] US 9,695,216 B2.
[18] P. Gagnon, 1996, Purification Tools for Monoclonal Antibodies, Validated Biosystems, Tucson, 296 pp.
[19] WO 2013/180647 A1.
[20] WO 2015/183180 A1.

### Summary

A method has been developed for reduction of host DNA contamination in mammalian cell culture that is orthogonal to, and extends the capability of, the traditional standard of treatment with DNA-lytic enzymes. It is also more effective than the method of flocculation by citric acid. The method is directed to AAV-containing cell culture harvests, cell lysates, or other AAV-containing preparations produced by cell culture.

According to the invention the method for removing chromatin from a cell culture harvest is comprising the steps:
- providing a cell culture harvest containing a desired biological product wherein the desired biological product is an adeno-associated virus,
- incubating the cell culture harvest in an aqueous medium at a pH value within the range of 3.0 to 4.0 wherein the aqueous medium comprises sodium chloride at a concentration in the range of 3.0 M up to saturation and
- separating the desired biological product from solids produced.

In the method of invention the desired biological product is an adeno-associated virus.

In another embodiment of the method of invention the harvest may be obtained from a culture of mammalian cells, insect cells, yeast cells, or bacterial cells.

In yet another embodiment of the method of invention the pH can be about 3.5 ± 0.3.

In a further embodiment of the method of invention the period of incubation can be up to 24 hours, in particular in the range of 30 minutes to 240 minutes, or 45 minutes to 90 minutes, or 55 minutes to 65 minutes.

In a still further embodiment of the method of invention the solids can be separated from the soluble product by sedimentation or filtration.

In another embodiment of the method of invention incubation can be performed in the presence of a solid-phase material bearing primary amine groups.

In yet another embodiment of the method of invention the primary amine-bearing solid phase can be in the form of loose particles or in the form of a chromatography device.

In still another embodiment of the method of invention the solid phase particles may be present in a volumetric proportion in the range of 1% to 10%, or 2.5% to 7.5%, or 4.5% to 5.0% relative to the volume of cell culture harvest in an aqueous medium.

In a further embodiment of the method of invention the primary amine-bearing solid phase can be in a form selected from the group consisting of columns packed with porous particles, columns packed with nanofibers, columns packed with monolithic materials, hydrogels, porous membrane filters, and depth filters.

In a still further embodiment of the method of invention the primary amine surface of the solid phase may be accompanied by chemical residues of alternative character, for example secondary amines, tertiary amines, quaternary amines, hydrophobic residues, hydrogen bonding residues, metal affinity residues, or combinations thereof.

In another embodiment of the method of invention the method can be performed in the presence of one or more salts, sugars, surfactants, divalent metal ions, allantoin, polyethylene glycols, flocculating agents, or combinations thereof.

In another embodiment of the method of invention is further comprising the steps of
- reducing ionic strength of a solution comprising the desired biological product
- removing further contaminants by contacting the solution with a cation exchanger, wherein the cation exchanger binds the contaminants
- further reducing ionic strength of the solution comprising the desired biological product
- purifying the desired biological product by contacting the solution with a cation exchanger, wherein the cation exchanger binds the desired biological product.

An "ionic strength corresponding to NaCl" is defined by comparing the conductivity of a solution of NaCl in pure water at the same temperature as the aqueous medium comprising the cell culture harvest. In a preferred embodiment, the aqueous buffer comprises 3.0 to saturation of NaCl.

"Chromatin" is the remnant of the chromosomal mass of the host cells. Its primary content are nucleosomes, consisting of DNA and proteins, especially histones.

### Description of The Figures

Figure 1 shows the influence of NaCl concentration on chromatin reduction at pH 3.5.
Figure 2 shows the improved performance by inclusion of primary amine-bearing particles.
Figure 3 shows the influence of pH in the presence of primary amine-bearing solid phase particles.
Figure 4 shows the effect of pH on AAV recovery at 5 M NaCl.
Figure 5 shows the results from cation exchange chromatography.
Figure 6 shows the anion exchange chromatography of AAV purified by DNA extraction and cation exchange chromatography.
Figure 7 shows the DNA extraction followed by preferential exclusion chromatography.
Figure 8 shows the analytical anion exchange chromatography of AAV2/8 after extraction with primary amine-bearing particles at pH 3.5 in the presence of 5.0 M NaCl.
Figure 9 shows the comparison of flocculation by citric acid with solid phase extraction on primary amine bearing particles at high salt.
Figure 10 shows the refinement of pH response.
Figure 11 shows the influence of salt concentration during solid phase extraction on cation exchange chromatography.
Figure 12 shows the effect of primary amine-bearing particles at various proportions.
Figure 13 shows the effects of different salt species.

### Detailed Description of The Invention

One feature of the method is its use of distinctive and unusually high salt concentrations such as from 3 M to saturation. Saturating concentrations vary from one species of salt to another. Sodium chloride (NaCl) saturates at concentration of about 5 M. The extreme salt concentrations are intended to promote dissociation of host DNA from histone proteins, thereby causing the host DNA to become soluble. To the extent that a subset of host-derived DNA may be bound to AAV capsid surfaces, the extreme conditions are intended to co-promote dissociation and solubilization of that particular subpopulation of DNA from the capsids. The approach of deliberate dissociation and solubilization of host DNA from pre-existing associations is noteworthy because it is counterintuitive to the task of removing it. In the field of flocculation, for example, the benefits are achieved by precipitating the contaminants since soluble contaminants remain with the soluble product.

Another feature of the method of the invention is that along with highly elevated concentration of salt, it simultaneously employs pH values in the range of pH 3.0 to pH 4.0. These conditions are believed to further favor dissociation of host DNA from pre-existing associations with other species, including histones, transcription factors, and potentially from AAV capsids. The combination of high salt concentration and low pH for reduction of host DNA from any biological product is unknown in the art. However, there is an unexpected feature of AAV response to pH at high salt that goes a further step and makes the most effective range completely unpredictable. It would be reasonable to speculate that the relationship between pH and performance of the method would follow a linear progression with recovery decreasing along with pH. Instead, experimental data reveal that in the presence of 5.0 M NaCl, AAV capsid recovery improves with decreasing pH to a maximum at pH 3.5, and then it plunges at pH 2.5, revealing an unexpected window of best performance in the narrow range to pH 3.0 to 4.0. see data in examples 3 and 4.

The approach of applying elevated salt concentrations within this narrow window is more effective by itself than other known methods for extracting chromatin from AAV preparations. Optional extensions further enhance its utility.

In addition to the narrow pH window at high salt concentration, the method may be optionally defined by its integration of a solid phase extraction material with a primary amine surface chemistry. A primary amine surface endows the solid phase with unexpectedly high affinity for DNA within the high salt and low pH conditions of the basic method. Amine-based solid phase surfaces are commonly classified as anion exchangers because they are positively charged. Anion exchangers are widely understood in the art to provide their best DNA binding characteristics at salt concentrations below 0.1 M and slightly alkaline pH values such as pH 8.0 to pH 8.5. Even within this pH range, anion exchangers are widely recognized for their inability to bind DNA at salt concentrations greater than about 0.4 to 0.6 M NaCl. Very surprisingly, we have found that the primary amine solid phase used to perform the method is able to bind DNA when exposed to a combination of high salt concentrations such as 3 M to saturation, and strongly acidic pH conditions such as from pH 3.0 to pH 4.0.

The final step of the method, with or without inclusion of the primary amine solid phase, is separation of the sample from solids produced by the method, while still under the conditions of 3 M to saturating salt and pH 3.0 to 4.0.

In addition to the pH, salt concentration, and presence or absence of the primary amine solid phase, and especially where there is a strong desire to reduce host DNA contamination to the lowest possible levels, the sample may optionally be treated with DNA-lytic enzymes. Such enzymes, the basic methods to use them, and methods to optimize their performance are known widely in the art, including in the field of AAV purification. Treatment with DNA-lytic enzymes may occur before or after the present method, or at any stage of a multistep purification sequence, for example after a tangential flow filtration step or after a chromatography step.

The reaction mixture may optionally contain a sugar such as sucrose in a concentration ranging from 1% to 10%. It may alternatively contain a sugar such as sorbitol over a range of concentrations from 2% to 20%, or mannitol or xylitol over a similar range of concentrations. It may alternatively contain other sugars or combinations of sugars. The inclusion of sugars is believed to favor stability of AAV capsids and may favor higher recovery of the product. Experimental data indicate that sugars do not significantly alter the degree of DNA extraction. In any such case, the sugar may be present either because it was in the original sample being treated or because it was added in conjunction with the present treatment.

The reaction mixture may optionally contain the surfactant Pluronic F68 at a concentration ranging from 0.01% to 1.00%. It may alternatively contain the surfactant Tween, or the surfactant Triton, or any other non-ionic or zwitterionic surfactant or combination of surfactants over a similar range of concentrations. The inclusion of surfactants is believed to aid in suppression of non-specific interactions that may lead to virus being lost by sticking to container and/or tubing walls and may favor higher recovery of the virus. Experimental data suggest that Pluronic F68 contributes modestly and indirectly to the ability of the method to extract DNA, perhaps through this mechanism. In any such case, a surfactant may be present either because it was in the original sample being treated or because it was added in conjunction with the present method.

The reaction mixture may optionally contain divalent metal cations such as magnesium ions. It may alternatively contain calcium ions. It may alternatively contain a combination of magnesium and calcium ions. It may alternatively contain other metal ions or combinations of ions. The inclusion of metal ions is believed to improve the stability of AAV capsids and may improve the recovery of the product. Experimental data suggest that metal ion addition contributes indirectly and modestly to the ability of the extraction method to remove host DNA. In any case, the metal ions may be present either because they were in the original sample being treated or because they were added in conjunction with the present treatment.

The reaction mixture may optionally contain allantoin at a concentration ranging from 1% to 10%, or 2% to 5%, or within a broader or narrower, higher or lower range. Experimental data indicate that allantoin does not contribute directly to extraction of DNA but may indirectly enhance overall performance of the method by reducing the content of turbidity-forming nanoparticulates that can impair filtration or chromatography methods. Allantoin may be added before or after other solids have been removed from the sample.

The reaction mixture may optionally contain nonionic organic polymers such as polyethylene glycol at a concentration of 0.5% to 5.0%.

The reaction mixture may optionally contain common flocculating agents of various character. One class of such flocculating agents includes fatty acids containing 6 to 10 carbon atoms at a concentration of 0.1% to 1.0%. The reaction mixture may alternatively and/or additionally contain anionic flocculating agents such as chitosan, or ethacridine, or methylene blue, or polyethyleneimine (PEI), or polydiallyldimethylammonium chloride (pDADMAC), or chlorhexidine, or benzalkonium chloride in amounts ranging from 0.01% to 0.1%. In any case, such flocculating agents may be present either because they were in the original sample being treated or because they were added in conjunction with the present treatment.

In a preferred embodiment, the method of the invention is followed by further purification steps.

It will be apparent that the high concentration of salt remaining after treatment will necessitate processing to make the sample conditions suitable for follow-on purification methods. The pH of the sample may be changed in conjunction with reducing salt concentration. Under some circumstances it may be expedient to achieve these changes by tangential flow filtration (TFF). Under some circumstances it may be expedient to achieve these changes by buffer exchange chromatography. Under some circumstances it may be expedient to achieve these changes by dialysis. Under some circumstances it may be expedient to achieve these changes by dilution. These options, how to select them, and how to perform them have been well known in the art for decades.

In some cases, it will be useful to alter the sample conditions in two steps rather than in a single step. In one illustrative but non-limiting example, the treated sample is diluted to a concentration of NaCl of about 1.0 M. The diluent in this case may be a buffer such as 50 mM formic acid with a pH of about 3.5. The sample is then contacted with a solid phase that has been chemically modified to present sulfo (SO₃) groups, such as chromatography particles, or a chromatography device, or a filtration device that have been pre-equilibrated to the same conditions. Under such conditions, AAV virus will not be retained but contaminants with higher affinity for the SO₃ groups may do so and by this means be eliminated from the sample. Such contaminants will particularly include histone proteins and other contaminants that carry a positive charge at the operating pH. Having now eliminated these contaminants from the sample, the sample may be diluted to a greater degree to enable the virus to be retained by a chromatography material, including but not limited to an SO₃ cation exchange material. It will be apparent that the easiest way to achieve the initial reduction of salt concentration is by simple dilution as described but may optionally be achieved by dialysis, TFF, or buffer exchange chromatography according to personal preference.

Experimental data suggest that in addition to removing host-derived DNA, the method also removes a large proportion of contaminating host-derived RNA and host-derived proteins. On a practical level, this provides the benefit of further improving the performance of follow-on purification steps. It is logical from a chemical perspective that RNA should be removed by a method that removes DNA because they bear many chemical similarities, but this does not explain how the method reduces protein contamination. One hypothesis is that histone proteins released from DNA precipitate under the reaction conditions, then due to their extreme hydrophobicity, they act as nucleation centers for secondary accretion of other protein species.

It will be apparent to persons of skill in the art that strong binding of DNA to the primary amine solid phase implies that it will support similarly strong binding of other highly phosphorylated contaminants including endotoxins, lipid-enveloped viruses, and extracellular vesicles such as exosomes, microvesicles, and apoptotic bodies; or viral or vesicular debris, along with organelles or membrane debris from the original host cells. Since high salt concentrations and low pH are known individually to be destructive to viruses and extracellular vesicles, the combination of these conditions with the ability of the solid phase to bind viruses and vesicles is expected to enhance elimination of both contaminant classes.

Higher concentrations of salts will generally promote more effective dissociation and solubilization of DNA from histones. Preliminary experimental data indicate that NaCl is most effective. To the extent that host DNA may be bound to the outer surfaces of AAV capsid proteins, the highest salt concentration should also promote its most effective dissociation from the outer capsid walls. As a general matter, the salt concentration should be the highest at which the AAV particles remain soluble. Salts of multivalent anions such as phosphate, citrate, and sulfate can produce positive results but they cause precipitation of AAV at concentrations greater than 1 M. They also weaken the attraction of DNA to the solid phase.

The method can be practiced at acidic pH values in the range of 3.0 to 5.0, or 3.5 to 4.5, or 3.3 to 3.7, or 3.4 to 3.6, or at pH 3.5, or at pH 3.5 ± 0.1, or at 3.5 ± 0.2, or at 3.5 ± 0.3, or at 3.5 ± 0.4, or at 3.5 ± 0.5. Low pH values create potential risk of damage to AAV particles but published studies in the field suggest that values down to pH 2.5 are tolerated for at least a few hours. Experimental data show clearly that practicing the method at pH values below 3.0 or above 4.0 reduce the effectiveness of the method, but still produce better results better than known alternatives for sample preparation.

The importance of buffer capacity for good process control is universally known in the art. Many resources are known for identifying suitable buffers for particular pH ranges, and their usage is well known by persons of skill in the art. To name a few without restricting the method, acetic acid, with a pKₐ of 4.71, is a suitable buffer for pH control over the range of 4.25 to 5.25, with good buffer capacity at pH 4.5. Formic acid, with a pKₐ of 3.77 is a suitable buffer for pH control over the range of 3.25 to 4.25, with good buffer capacity at pH 3.5 to 3.75. Glycine, with a pKa of 2.96 is a suitable buffer for pH control over the range of 2.5 to 3.5 with good buffer capacity at pH 3.0. Alanine, with a pKa of 2.34 is a suitable buffer for pH control over the range of 1.8 to 2.8 with good buffer capacity at pH 2.0 to pH 2.5. A combination of glycine (pKa 2.96) titrated to pH 3.5 ± 0.5 with formic acid (pKa 3.77) represents an especially suitable combination for providing good buffer capacity and pH control in this range.

The concentration of buffer should be sufficient to ensure that the pH of the reaction mixture is as close as possible to the selected target pH. Depending on the content and buffering characteristics of the sample, the concentration of the buffer may be in the range of 10 mM to 500 mM, or 20 mM to 200 mM, or 50 mM to 100 mM. Selecting an appropriate buffer concentration is a routine and simple matter well known throughout the art of purifying biological products. A convenient starting concentration is about 100 mM since this concentration will overwhelm the native buffering characteristics of most samples to be processed by the method. So long as target pH is achieved, higher or lower concentrations of buffer will not affect the results obtained by the method.

The period of incubation after achieving the target high salt low pH conditions may be 15 minutes, or 30 minutes, or 60 minutes, or 90 minutes, or 120 minutes, or more, or less, or an intermediate period of time. Experimental results show that an incubation period of 60 minutes achieves results very similar to 120 minute incubation. Longer incubation periods may promote more complete dissociation of DNA from histones and/or more complete DNA binding to the solid phase. Shorter intervals may be more convenient. Given the known extensive variability among AAV production media, it is within the norms of the art to consider a range of incubation periods to identify the incubation time supports the best results for any given sample.

In one embodiment, the method is performed on a cell culture harvest in which the cells secrete AAV particles into the extracellular cell culture medium. In another embodiment, the method is performed on a cell lysate, in which AAV-containing cells have been treated to release the AAV particles into the surrounding medium. In another embodiment, the method is performed on a partially purified preparation containing an excess of DNA.

In some embodiments the incubation includes a solid phase, especially a solid phase with primary amines.

In one operating format, the primary amine-bearing solid phase is in the form of particles which are present in a mixture with an AAV cell culture harvest or cell lysate at high salt and low pH. In another operating format, the primary amine-bearing solid phase is in the form of a device through which is passed the AAV cell culture harvest or cell lysate at high salt and low pH. In another operating format, the primary amine bearing solid phase is in the form or particles which are present in the mixture with an AAV cell culture harvest or cell lysate at high salt and low pH and, after removal of the solid phase, the supernatant is passed through a primary amine-bearing solid phase in the form of a chromatography device.

It will be apparent that each of the operating formats presents features that may be viewed as relative strengths or weaknesses. The particle-only format provides the most convenience since it requires no additional steps before conducting a follow-on chromatography or other purification step. The solid-phase device provides higher surface contact efficiency than the particles, which should translate to shorter contact time and DNA removal to lower levels. A combination of the two formats may be more effective than either one alone.

Any solid phase particles with a primary amine surface chemistry may be used to practice the method. Solid phase particles may be nonporous or they may contain pores and/or channels that permit diffusive and/or convective entry of molecules up to 1 µm in size. Experimental data show that neither the size nor porosity of the particles contributes substantially to the effectiveness of the treatment. The key feature is that they provide a solid phase surface to present the primary amine chemistry to the reaction solution. Particles size may accordingly range from 5 nm to 500 nm, or 10 nm to 400 nm, or 40 nm to 300 nm, or 80 nm to 200 nm, or 100 nm to 150 nm, or some other interval within the range of 5 nm to 500 nm. Average pore and/or channel size may range from less than 1 nm to 10,000 nm, or 10 nm to 7,500 nm, or 100 nm to 5000 nm, or 1000 nm to 2000 nm, or any other interval from less than 1 nm to 10,000 nm.

Solid phase particles may be added at a volumetric proportion of about 1% of the sample volume, or 2% of the sample volume, or 3% of the sample volume, or 4% of the sample volume, or 5% of the sample volume, or 10% of the sample volume, or a larger or different proportion. It will be understood by persons of skill in the art that they amount of DNA in various AAV preparation may vary considerably. Since the overall method is directed particularly to DNA removal, simple routine experimentation may be required to determine the proportion of solid phase particles that most effectively removes DNA. Using an excess amount, such as 5% will typically suspend the need to perform such experiments but process economic considerations may make such experiment desirable since they may demonstrate effectivity of lesser amounts.

Solid phase extraction devices used to perform the method may consist of columns packed with porous particles, columns packed with nanofibers, columns packed with monolithic materials, hydrogels, membrane filters, and depth filters of any format or configuration.

The surface of the solid phases in any physical format is chemically modified to have at least one primary amine covalently attached to its surface. It may contain ligands consisting only of single primary amines, or polymers of primary amines, or combinations of primary amines with secondary amines, or tertiary amines, or quaternary amines, or any combination of amines. The solid phase may additionally bear other chemical moieties, including but not limited to hydrophobic moieties, hydrogen bonding moieties, metal binding moieties, and negatively charged moieties. Solid phases lacking primary amines may also be present with solid phase particles bearing primary amines.

As a matter of good process control, the solid phase, whether in the form of loose particles or in a chromatography device, should be equilibrated to the reaction conditions before they are contacted with the sample.

Removal of solids may be achieved by any convenient means. Many such methods and the criteria for selecting them are widely known in the art. Any of such means may be used without altering the ability of the method to achieve its express purpose of reducing the amount of DNA in a preparation of AAV.

In one embodiment, solids created by the method may be removed by gravitational sedimentation.

In another embodiment, sedimentation of solids may be accelerated by centrifugation.

In another embodiment, sedimentation of solids may be accelerated by acoustic waves.

In another embodiment, sedimentation of solids may be achieved while the mixture is pumped in an ascending direction through a cylinder, allowing denser sediments to settle by gravity while the solid-deficient carrier liquid containing the virus passes up and out of the cylinder. In one such embodiment, the liquid may be passed through a filtration device after its passage out of the cylinder.

In another embodiment where solids are not removed by sedimentation, they may be removed by filtration.

In another embodiment, a filtration method used to remove solids after treatment may be a membrane filtration method or a depth filtration method.

In another embodiment, removal of solids may be aided by the use of a socalled bag filter, or sock filter, in which a porous membrane is configured as a cylinder with an open end to admit the sample, but with the other end closed, so that liquid is forced through the pores.

A non-limiting example is given to illustrate the basic steps in performing the method. Primary amine-bearing particles are equilibrated in advance to 100 mM formic acid, 4 M NaCl, pH 3.5. A defined volume of a cell lysate or cell culture harvest is placed in an appropriate vessel. Dry salt such as sodium chloride is added in an amount sufficient to give a final concentration 4 M, and pH is adjusted to 3.5 by titration with formic acid. The solid phase particles may be present already when the sample conditions are adjusted, or they may be added after the conditions are adjusted. In either case, the particles are present at a volumetric proportion of about 5% of the original sample volume. The combination is permitted to incubate mixing for a period of 60 minutes at ambient temperature. Solids, including the solid phase extraction particles, are then removed by any convenient means including but not limited to centrifugation or filtration. For emphasis, solids must be removed while they are still resident in the working solution.

Another non-limiting sample is given to illustrate the basic steps in performing the method. Primary amine bearing particles are equilibrated in advance to 100 mM formic acid, 5 M NaCl, pH 3.5. A defined volume of a cell lysate or cell culture harvest is placed in an appropriate vessel. Another vessel is filled with the same volume of a two-fold-concentrated reagent mixture, containing sodium chloride sufficient to produce a concentration of 5 M when diluted with the sample; and containing formic acid, pH 3.5 sufficient to produce 100 mM formic acid pH 3,5 when diluted with the sample; and containing primary amine-bearing particles sufficient to produce a volumetric proportion of 5% when diluted with the sample. The sample is added to the concentrated reagent and permitted to incubate mixing for a period of 60 minutes at ambient temperature. Solids produced by exposure of the sample to high salt and low pH are then removed by any convenient method including but not limited to centrifugation or filtration.

Materials and instructions for performing the method may be provided in the form of kits. In one embodiment, a kit provides solid phase particles in concentrated buffer in a prefilled vessel to which the end user will add sample and incubate. The incubated sample will be processed with a provided centrifugal spin cartridge employing a 0.22 µm membrane to remove solids from the AAV-containing liquid. One version of the kit will contain enough materials to perform purification of 10 purifications of 10 mL each. Alternative versions may be configured to process smaller or larger sample volumes, and/or with materials sufficient to perform fewer or more purifications. An extended version of the kit may include one or more chromatography devices to conduct purification. A further extended version may include prepared tubes containing a cell lysis buffer.

In an alternative embodiment, not forming part of the invention, the kit will include a buffer concentrate lacking a particulate solid phase in a prefilled container, to which the end user will add sample and incubate for an appropriate period of time. The incubated sample will be processed with a provided centrifugal spin cartridge employing a 0.22 µm membrane to remove solids from the AAV-containing liquid. The liquid will then be passed through a provided reusable chromatography device containing the solid phase to extract DNA according to the method. One version of the kit will contain enough materials to perform purification of 10 purifications of 10 mL each. Alternative versions may be configured to process smaller or larger sample volumes, and/or with materials sufficient to perform fewer or more purifications. An extended version of the kit may include one or more chromatography devices to conduct purification. A further extended version may include prepared tubes containing a cell lysis buffer.

In a further alternative embodiment that requires no chromatography, the kit will include a buffer concentrate with the solid phase in a prefilled container, to which the end user will add sample and incubate for an appropriate period of time. The incubated sample will be optionally processed with a provided centrifugal spin cartridge employing a 0.22 µm membrane to remove solids from the AAV-containing liquid. The sample will then be processed with a provided centrifugal cartridge employing a 300,000 MWCO (molecular weight cutoff) membrane which will retain high molecular weight contaminants while permitting the virus to pass through the membrane. The sample will then be processed with a provided centrifugal cartridge employing a 100,000 MWCO membrane which will the virus while permitting low molecular weight contaminants to pass through the membrane. This step will also concentrate the virus and enable its buffer exchange into a final formulation.

The invention is further illustrated by the following, non-limiting examples.

### Examples

### Example 1

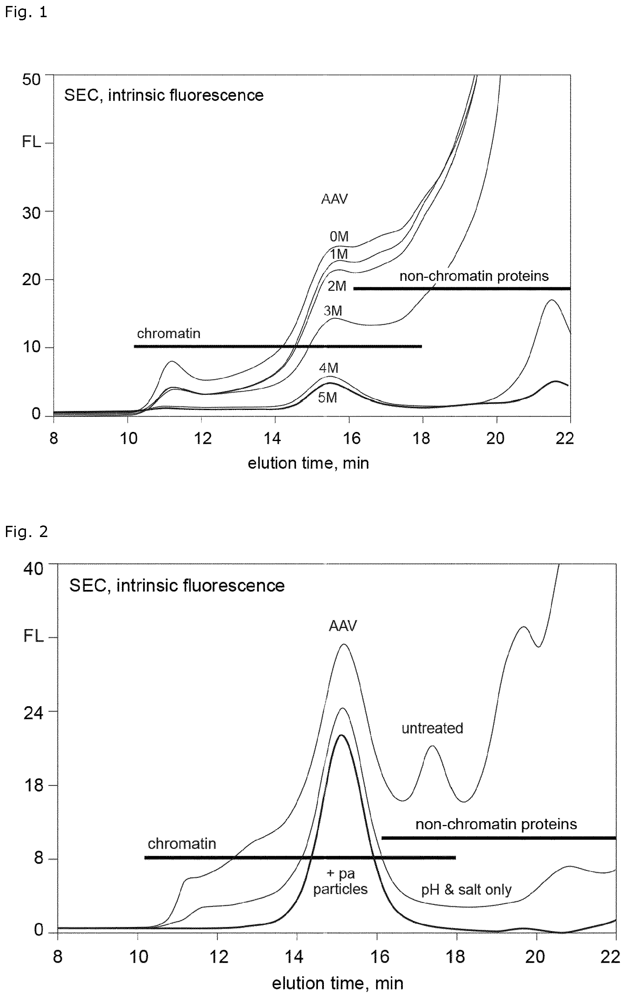

The influence of NaCl concentration on chromatin reduction at pH 3.5. Figure 1 illustrates results from an experiment at pH 3.5 with different concentrations of salt ranging from 0 M to 5 M. The analytical method is size exclusion chromatography with fluorescence monitoring to detect the intrinsic fluorescence of tryptophan residues in proteins. This method amplifies the sensitivity and enables detection of the AAV capsids in the sample. Samples were prepared by titrating the pH by performing a 1:1 volumetric dilution with 200 mM formic acid, pH 3.5 and twice the amount of salt necessary to produce the target concentration. Experimental results showed increasing reduction of chromatin with increasing NaCl concentration. AAV remained soluble throughout the range. Salt concentrations of 1 M and 2 M reduced chromatin contamination by perhaps 40% but had little effect on reducing contamination by non-chromatin proteins. 3 M was little more effective with chromatin reduction but showed a significant improvement in reduction of non-chromatin proteins. 4 M NaCl showed at least a 90% reduction of chromatin and an even greater reduction of non-chromatin proteins, leaving the AAV soluble. The effect of 5 M NaCl was slightly better for contaminant reduction but may have slightly reduced recovery of AAV.

### Example 2

Superior performance with by inclusion of primary amine-bearing particles. The ability of primary amine-bearing particles to contribute to a better overall result was evaluated. One sample was equilibrated to 5.0 M NaCl, 50 mM formic acid, 5 mM MgCl₂, and 0.1% Pluronic F68, pH 3.5. Another was equilibrated to the same conditions except including 5% primary amine bearing particles. Both samples were incubated for 1 h at room temperature, after which solids were removed by membrane filtration. Results are compared with the untreated control in Figure 2. The conditions lacking the particles reduced chromatin content by about 40% and reduced non-chromatin proteins by a very substantial amount not estimable from the figure. Inclusion of the particles reduced chromatin by more than 95% and also reduced non-chromatin proteins to a far greater extent. AAV recovery was about the same with or without particles.

### Example 3

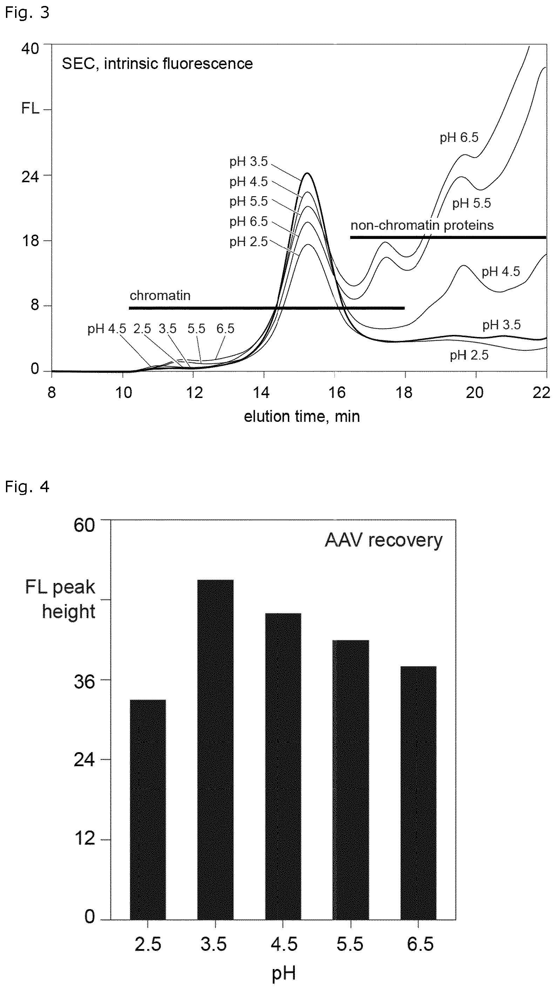

Improvement of chromatin clearance as a function of pH in the presence of primary amine-bearing solid phase particles. Figure 3 shows that inclusion of primary amine particles and the influence of pH on removal of chromatin and other contaminants while the AAV remains soluble. All experiments were performed at 5 M NaCl, in the presence of 5% primary amine-bearing particles, 100 mM formic acid, 5 mM MgCl₂, and 0.1% Pluronic F68 (surfactant). In combination with example 1, these results emphasize that high salt concentration is the master variable but show that pH makes a substantial contribution to overall efficacy. The best virus recovery is observed at pH 3.5, which also supports the lowest chromatin and non-chromatin protein content with pH 2.5. However, pH 2.5 gives the lowest AAV recovery. Recovery at pH 4.5 is nearly as good as pH 3.5, but chromatin reduction is modestly inferior and non-chromatin protein contamination is 2-3 times higher than at pH 3.5. Results at pH 5.5 and 6.5 are inferior in all respects. The higher AAV recovery at pH 3.5 recommends this pH in particular for practicing the method. In separate experiments comparing formic acid and glycine as buffers, results with formic acid were clearly superior. In other experiments, 5 mM MgCl₂ was observed to make a small but clear positive contribution to reduction of contaminants while maintaining high virus recovery. In another series of experiments, incubation periods of 1 h and 2 h were compared. 1 h gave the same results as 2 h.

### Example 4

### DNA extraction followed by cation exchange chromatography

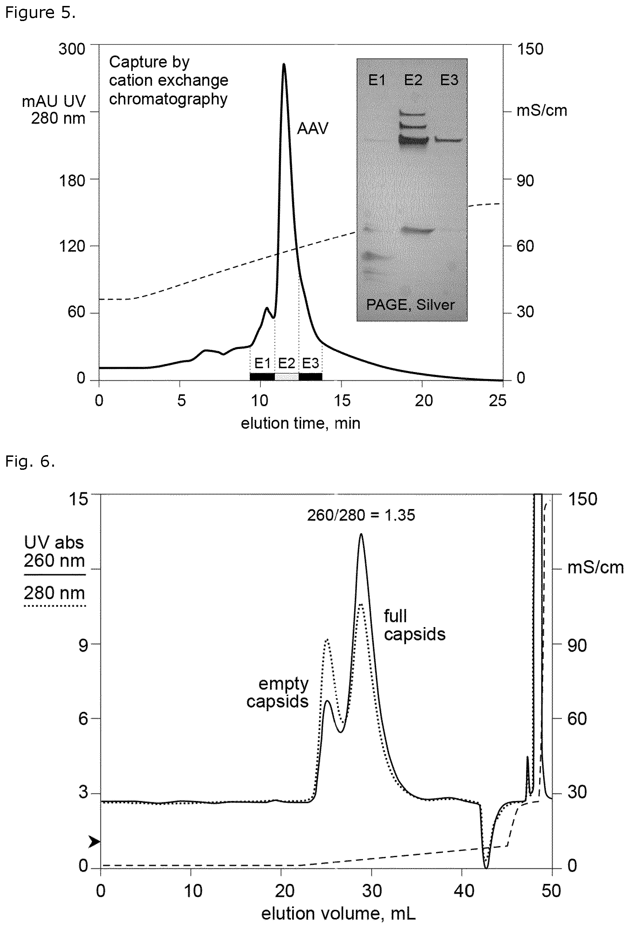

AAV-containing insect cell lysate was equilibrated to 100 mM formic acid, 5.0 M NaCl, 5 mM MgCl₂, 0.1% Pluronic F68, 5% primary amine-bearing particles, pH 3.5. The mixture was incubated at room temperature for 1 h and the solids removed by membrane filtration. The supernatant was diluted by addition of 50 mM formic acid, pH 3.5 until conductivity reached 40 mS/cm. A cation exchange monolith (CIMmultus SO3, BIA Separations) was equilibrated to the same conditions and the sample was loaded. As shown in the Figure 4, a concentrated peak containing greater than 90% pure AAV was obtained. Figure 4 plots AAV recovery as a function of pH and highlights two major unexpected findings. It was expected that recovery would be highest at the pH closest to the physiological environment in which the virus normally exists. Indeed the lowest pH, of 2.5, gave the lowest recovery of all pH values. The first unexpected finding was that the second lowest recovery was observed at pH 6.5, the closest value to physiological, and that recovery increased from pH 6.5 to pH 3.5. The second unexpected finding was that the maximum virus recovery was observed at pH 3.5. These findings highlight the unpredictable nature of the system at high salt concentrations, in this case at 5 M NaCl. The chromatogram is shown in Figure 5, with an inset showing a PAGE gel stained with silver.

### Example 5

### DNA extraction followed by cation exchange chromatography then anion exchange chromatography

The sample processed by DNA extraction and cation exchange chromatography in Example 4 was further processed by anion exchange chromatography (CIMmac AAV, BIA Separations) to illustrate the DNA extraction in the context of a complete purification process. In brief, fractions E2 and E3 (see Figure 5) were pooled, the pH was adjusted to 9.0 and the sample was diluted to a conductivity of about 2.5 mS/cm. The column was equilibrated to 50 mM bis-tris-propane, pH 9.0, and the sample was loaded onto the column. The column was washed with 5 CV of equilibration buffer then eluted with a 20 CV linear gradient ending at 50 mM bis-tris-propane, 200 mM NaCl, pH 9.0. The elution profile is illustrated in Figure 6. It shows the characteristic two-peak AAV profile where the first peak corresponds to empty capsids and the second peak corresponds to vector-DNA-filled capsids. The capsid proteins are transparent to UV, so the internal DNA contributes to excess 260 absorbance by the full capsids. The empty capsids, lacking internal DNA, give 260/280 absorbance more typical of proteins. The ratio of absorbance at 260 nm and 280 nm is considered an indicator of AAV purity, with a value of 1.30 or below indicating an impure preparation. Note the ratio in the full capsids is about 1.35 in this case, indicating high purity. This is attributed to the effects of the DNA extraction step enhancing the purification performance of both the cation exchange and anion exchange chromatography steps.

### Example 6

### DNA extraction followed by preferential exclusion chromatography

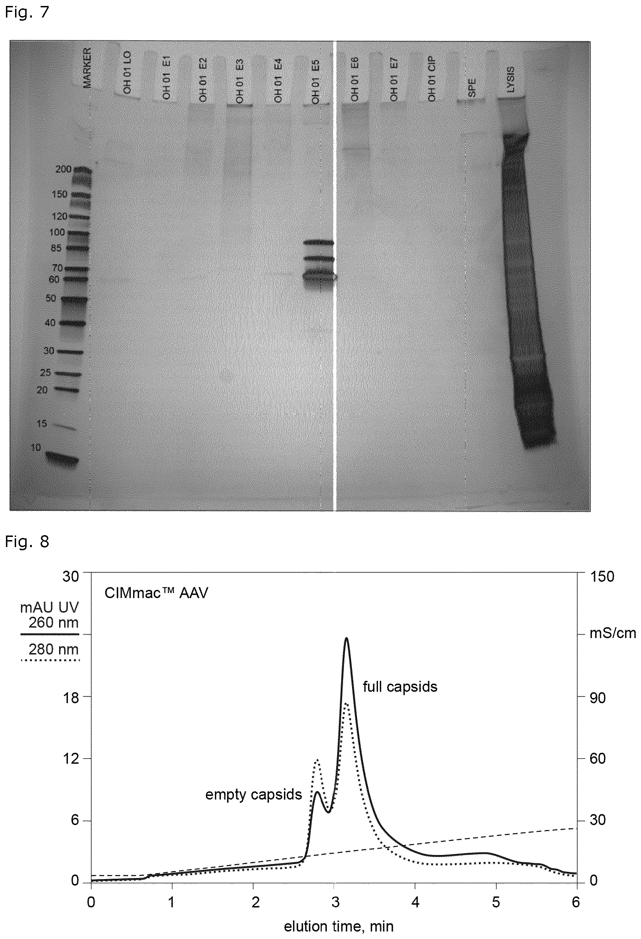

AAV-containing insect cell lysate was equilibrated to 100 mM formic acid, 5.0 M NaCl, 5 mM MgCl₂, 5% primary amine-bearing particles, pH 3.5. The mixture was incubated at room temperature for 1 h and the solids removed by membrane filtration. The supernatant was diluted 1:1 with 3.0 M potassium phosphate, pH 7.0. and applied to a CIMmultus OH monolith (BIA Separations) that had been previously equilibrated to 1.5 M potassium phosphate, pH 7.0. After sample loading, the column was washed with equilibration buffer to displace unbound contaminants from the column. The AAV was eluted with a descending linear salt gradient ending at 50 mM potassium phosphate buffer. Figure 7 illustrates results from polyacrylamide gel electrophoresis (PAGE) analysis (silver staining) of the column fractions. The original treated sample is in the last well at the far right. The next well to the left shows the sample after DNA extraction. At the far left are the molecular weight markers, then the DNA extracted sample diluted and ready to load on the column. The other wells show the contents of fractions collected during the chromatography run. The contents of the elution peak are shown at the middle of the gel with the label OH 01 E5.

Note that the AAV bands are very intensely stained but contaminant staining is remarkably low, indicating a high degree of purity.

### Example 7

### DNA extraction followed by differential membrane filtration

AAV-containing insect cell lysate was equilibrated to 100 mM formic acid, 5.0 M NaCl, 5 mM MgCl₂, 0.1% Pluronic F68, 5% primary amine-bearing particles, pH 3.5. The mixture was incubated for 1 h at room temperature and the solids removed by membrane filtration. The sample was titrated to a pH of 6.5 ± 0.5, then applied to a centrifugal membrane filtration device with a molecular weight cutoff of 300,000 kDa to retain residual high molecular weight chromatin, such as seen in the sample from example 4. The virus passed through the filter membrane and was them applied to a centrifugal membrane filtration device with a molecular weight cutoff of 100,000 kDa to retain the virus while permitting low molecular weight contaminants to pass through the membrane. The initial sample volume was restored by addition of 50 mM Hepes, 50 mM NaCl, 50 mM arginine, 1% sorbitol, 2 mM magnesium chloride, pH 7.0, and recentrifuged. This left the sample concentrated about 20-fold and in the new buffer. The product was evaluated by analytical anion exchange chromatography. Results are show in Figure 8. Compare with Figure 6 for reference.

### Example 8

### Comparison of flocculation by citric acid with solid phase extraction on primary amine bearing particles at high salt

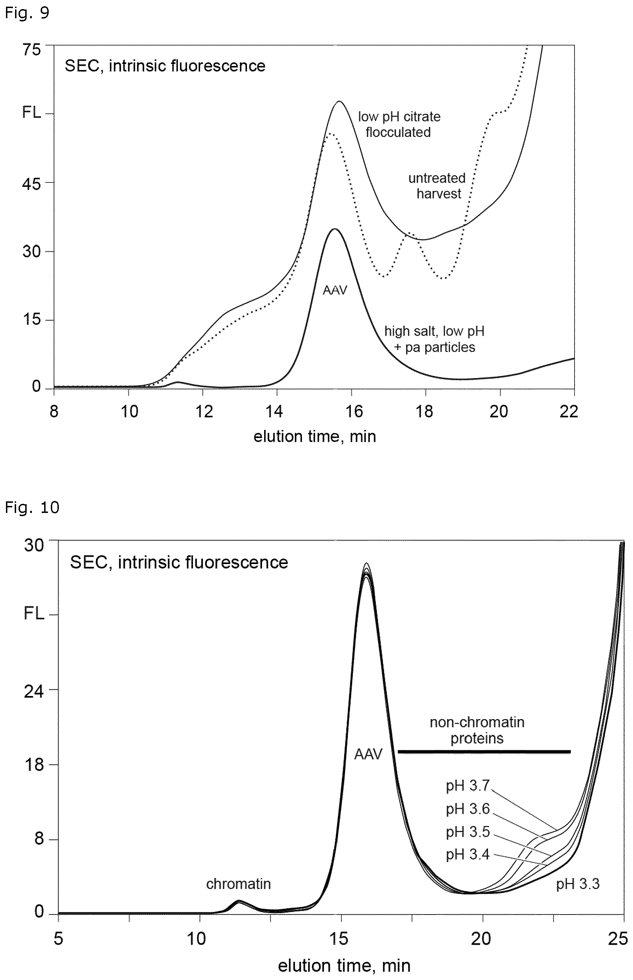

Filtered AAV-containing harvest from SF9 cells was processed by the citric acid flocculation method described by Potter and Byrne [6] to highlight the relative performance of the present invention. Citric acid was added to a final concentration of 20 mM and the pH of the preparation titrated to pH 3.5, relying on the added citrate to provide buffering. In parallel, formic acid (100 mM), magnesium chloride 95 mM), Pluronic F68 (0.1%), and primary amine-bearing particles (5% v:v) were added to another aliquot of filtered harvest and then titrated to a pH of 3.5. Both treatments were incubated for 60 minutes before removing solids by filtration. Few visible solids were observed with the citric acid flocculation treatment. Results are compared in Figure 9. As shown, the contaminant profile after citric acid flocculation was worse than before treatment, and dramatically inferior to treatment with high salt and primary amine-bearing particles.

### Example 9

### Refinement of pH response

Examples 1 and 3 demonstrated that the optimum pH was about 3.5±0.5 in the absence of primary amine-bearing particles. An additional series of experiments was conducted to refine the estimation of pH optimum in the presence of primary amine-bearing particles. Filtered AAV-containing harvest was treated with 5.0 M sodium chloride, 5 mM magnesium chloride, 1% Pluronic F68, 5% v:v primary amine-bearing particles. Samples were titrated to pH 3.3, 3.4 3.5, 3.6, and 3.7 respectively. They were incubated for 60 min, the solids removed by filtration, and the samples were analyzed by size exclusion chromatography with monitoring by intrinsic fluorescence. As shown in Figure 10, the pH providing the most effective contaminant reduction without apparent loss of AAV particles was pH 3.3, with increasing contamination at higher pH values.

### Example 10

### Alternative demonstration of the utility of extreme salt concentrations

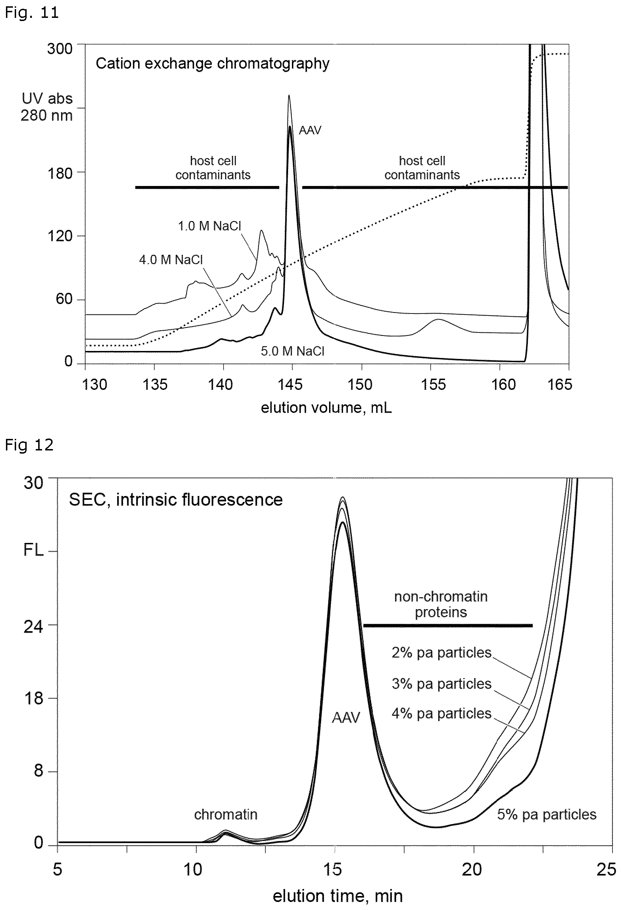

Example 1 showed that an increasing proportion of contaminants was removed with increasing sodium chloride concentration in the absence of primary amine-bearing particles, with 4 M NaCl providing lower contamination than 1 M, and 5 M providing significantly lower contamination than 4 M. A series of experiments was conducted to demonstrate that the effect of NaCl remained in the presence of primary amine-bearing particles and to demonstrate it by means of an alternative analytical format. In the new series of experiments, the influence of salt concentration during solid phase extraction was determined by fractionating the treated samples on a cation exchanger. Samples were prepared at pH 3.5 in the presence of 50 mM formic acid, 5 mM magnesium chloride, 1% Pluronic F68, 5% primary amine-bearing particles, and sodium chloride concentrations at 1 M, 4 M, and 5 M. Results are shown in Figure 11. The profile corresponding to solid phase extraction at 1 M NaCl shows heavy contamination leading to the main AAV peak. Contaminant levels are strongly reduced after solid phase extraction with 4 M NaCl, and the lowest levels are achieved after solid phase extraction at 5 M NaCl. According to these results, extreme salt concentrations are even more beneficial in the presence of primary amine-bearing particles than in their absence (Example 1).

### Example 11

### Proportional response to primary amine-bearing particles

A series of experiments was conducted to determine the most favorable proportion of primary amine-bearing. Filtered AAV2/8-containing cell culture harvest was treated with 50 mM formic acid, 5.0 M sodium chloride, 5 mM magnesium chloride, 1% Pluronic F68, and primary amine-bearing particles in volumetric proportions of 2%, 3%, 4%, and 5%, at pH 3.5. The mixtures were incubated for 60 minutes then solids were removed by membrane filtration. The samples were analyzed by size exclusion chromatography monitored by intrinsic fluorescence. As shown in Figure 12, increasing particle proportions produced measurably better contaminant reduction with a significant improvement at 5%.

### Example 12

### Effects of different salt species

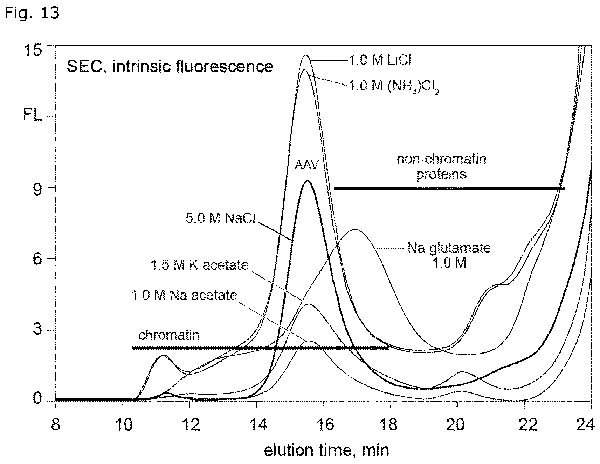

Example 1 showed that increasing levels of sodium chloride increased the utility of the method with respect to removing contaminants while conserving AAV. Additional species of salt were surveyed to assess their relative utility. All experiments were conducted with the same filtered AAV2/8 harvest from SF9 cells used for other experiments. All experiments were conducted with 50 mM formic acid at pH 3.5 in the presence of 5 mM magnesium chloride, 1% Pluronic F68, and 5% primary amine-bearing particles. Lithium chloride was added in one experiment to a final concentration of 1.0 M. Ammonium chloride was added in another to 1.0 M. Sodium glutamate was added in another to a final concentration of 1.0 M. Sodium acetate was added in another to a final concentration of 1.0 M, and potassium acetate in another to 1.5 M. All were compared to an experimental control with 5 .0 M sodium chloride. As shown in Figure 13, and comparing Figure 13 to Figure 1, sodium acetate and potassium acetate gave results similar to but better than sodium chloride at a similar concentration. Reduction of chromatin and non-chromatin proteins was outstanding with both and suggests that higher concentrations of these salts might offer utility equal to or greater than sodium chloride. However, apparent recovery of AAV appeared to be lower. Lithium chloride and ammonium chloride appeared to offer outstanding AAV recovery and better reduction of non-chromatin proteins than sodium chloride but inferior reduction of chromatin proteins. Sodium glutamate also gave better reduction of protein contamination than sodium chloride at the same concentration but was clearly inferior to all other salts in this concentration range. Beyond suggesting further exploration of salts other than sodium chloride, these results suggest the potential utility of combinations of two or more salts.

## Claims

1. A method for removing chromatin from a cell culture harvest comprising the steps:
- providing a cell culture harvest containing a desired biological product wherein the desired biological product is an adeno-associated virus,
- incubating the cell culture harvest in an aqueous medium at a pH value within the range of 3.0 to 4.0 wherein the aqueous medium comprises sodium chloride at a concentration in the range of 3.0 M up to saturation and
separating the desired biological product from solids produced.

2. The method of claim 1 wherein the harvest is obtained from a culture of mammalian cells, insect cells, yeast cells, or bacterial cells.

3. The method of any one of the claims 1 to 2 wherein the pH is 3.5 ± 0.3.

4. The method of any one of the claims 1 to 3 wherein the period of incubation is up to 24 hours, preferably in the range of 30 minutes to 240 minutes, or 45 minutes to 90 minutes, or 55 minutes to 65 minutes.

5. The method of any one of the claims 1 to 4 wherein the solids are separated from the soluble product by sedimentation or filtration.

6. The method of any one of the claims 1 to 5 wherein incubation is performed in the presence of a solid-phase material bearing primary amine groups.

7. The method of claim 6 wherein the primary amine-bearing solid phase in the form of loose particles or in the form of a chromatography device.

8. The method of claims 6 or 7 wherein solid phase particles are present in a volumetric proportion in the range of 1% to 10%, or 2.5% to 7.5%, or 4.5% to 5.0% relative to the volume of cell culture harvest in an aqueous medium.

9. The method of claims 6 or 7 wherein the primary amine-bearing solid phase is in a form selected from the group consisting of columns packed with porous particles, columns packed with nanofibers, columns packed with monolithic materials, hydrogels, porous membrane filters, and depth filters.

10. The method of any one of the claims 6 to 9, wherein the primary amine surface of the solid phase is accompanied by chemical residues of alternative character, for example secondary amines, tertiary amines, quaternary amines, hydrophobic residues, hydrogen bonding residues, metal affinity residues, or combinations thereof.

11. The method of any one of the claims 1 to 10, wherein the method is performed in the presence of one or more salts, sugars, surfactants, divalent metal ions, allantoin, polyethylene glycols, flocculating agents, or combinations thereof.

12. The method of any one of the claims 1 to 11, further comprising the steps of
- reducing ionic strength of a solution comprising the desired biological product
- removing further contaminants by contacting the solution with a cation exchanger, wherein the cation exchanger binds the contaminants
- further reducing ionic strength of the solution comprising the desired biological product
- purifying the desired biological product by contacting the solution with a cation exchanger, wherein the cation exchanger binds the desired biological product.

## Patentansprüche

1. Verfahren zur Entfernung von Chromatin aus einer Zellkulturernte, umfassend die Schritte:
- Bereitstellen einer Zellkulturernte, enthaltend ein gewünschtes biologisches Produkt, wobei das gewünschte biologische Produkt ein adenoassoziiertes Virus ist,
- Inkubieren der Zellkulturernte in einem wässrigen Medium mit einem pH-Wert innerhalb des Bereichs von 3,0 bis 4,0, wobei das wässrige Medium Natriumchlorid in einer Konzentration im Bereich von 3,0 M bis zur Sättigung umfasst, und
Trennen des gewünschten biologischen Produkts von produzierten Feststoffen.

2. Verfahren nach Anspruch 1, wobei die Ernte aus einer Kultur von Säugetierzellen, Insektenzellen, Hefezellen oder Bakterienzellen erhalten wird.

3. Verfahren nach einem der Ansprüche 1 bis 2, wobei der pH-Wert 3,5 ± 0,3 ist.

4. Verfahren nach einem der Ansprüche 1 bis 3, wobei die Inkubationsperiode bis zu 24 Stunden beträgt, vorzugsweise im Bereich von 30 Minuten bis 240 Minuten oder 45 Minuten bis 90 Minuten oder 55 Minuten bis 65 Minuten liegt.

5. Verfahren nach einem der Ansprüche 1 bis 4, wobei die Feststoffe von dem löslichen Produkt durch Sedimentation oder Filtration getrennt werden.

6. Verfahren nach einem der Ansprüche 1 bis 5, wobei eine Inkubation in Gegenwart eines Festphasenmaterials, das primäre Amingruppen trägt, durchgeführt wird.

7. Verfahren nach Anspruch 6, wobei die primäres Amin tragende Festphase in der Form von losen Partikeln oder in der Form einer Chromatographievorrichtung ist.

8. Verfahren nach den Ansprüchen 6 oder 7, wobei Festphasenpartikel in einem volumetrischen Anteil im Bereich von 1 % bis 10 % oder 2,5 % bis 7,5 % oder 4,5 % bis 5,0 % vorliegen, bezogen auf das Volumen der Zellkulturernte in einem wässrigen Medium.

9. Verfahren nach den Ansprüchen 6 oder 7, wobei die primäres Amin tragende Festphase in einer Form ist, die aus der Gruppe bestehend aus mit porösen Partikeln gepackten Kolonnen, mit Nanofasern gepackten Kolonnen, mit monolithischen Materialien gepackten Kolonnen, Hydrogelen, porösen Membranfiltern und Tiefenfiltern ausgewählt ist.

10. Verfahren nach einem der Ansprüche 6 bis 9, wobei die primäre Aminoberfläche der Festphase von Chemikalienresten von alternativer Beschaffenheit, beispielsweise sekundären Aminen, tertiären Aminen, quartären Aminen, hydrophoben Resten, Wasserstoffbrückenbildungsresten, Metallaffinitätsresten oder Kombinationen davon begleitet wird.

11. Verfahren nach einem der Ansprüche 1 bis 10, wobei das Verfahren in Gegenwart eines oder mehrerer Salze, Zucker, Tenside, zweiwertigen Metallionen, Allantoin, Polyethylenglykolen, Flockungsmitteln oder Kombinationen davon durchgeführt wird.

12. Verfahren nach einem der Ansprüche 1 bis 11, ferner umfassend die Schritte:
- Senken der Ionenstärke einer Lösung, umfassend das gewünschte biologische Produkt
- Entfernen weiterer Kontaminanten durch Inkontaktbringen der Lösung mit einem Kationentauscher, wobei der Kationentauscher die Kontaminanten bindet
- weiteres Senken der Ionenstärke der Lösung, umfassend das gewünschte biologische Produkt
- Aufreinigen des gewünschten biologischen Produkts durch Inkontaktbringen der Lösung mit einem Kationentauscher, wobei der Kationentauscher das gewünschte biologische Produkt bindet.

## Revendications

1. Procédé pour éliminer la chromatine dans une récolte de culture cellulaire, comprenant les étapes de :
- obtention d'une récolte de culture cellulaire contenant un produit biologique souhaité, lequel produit biologique souhaité est un virus adénoassocié,
- incubation de la récolte de culture cellulaire dans un milieu aqueux à une valeur de pH située dans la plage allant de 3,0 à 4,0, lequel milieu aqueux comprend du chlorure de sodium à une concentration située dans la plage allant de 3,0 M à la saturation, et
- séparation du produit biologique souhaité d'avec les solides produits.

2. Procédé selon la revendication 1, dans lequel la récolte est obtenue à partir d'une culture de cellules de mammifère, de cellules d'insecte, de cellules de levure, ou de cellules bactériennes.

3. Procédé selon l'une quelconque des revendications 1 et 2, dans lequel le pH est de 3,5 ± 0,3.

4. Procédé selon l'une quelconque des revendications 1 à 3, dans lequel la période d'incubation va jusqu'à 24 heures, de préférence est située dans la plage allant de 30 minutes à 240 minutes, ou de 45 minutes à 90 minutes, ou de 55 minutes à 65 minutes.

5. Procédé selon l'une quelconque des revendications 1 à 4, dans lequel les solides sont séparés du produit soluble par sédimentation ou filtration.

6. Procédé selon l'une quelconque des revendications 1 à 5, dans lequel l'incubation est effectuée en présence d'un matériau en phase solide portant des groupes amine primaire.

7. Procédé selon la revendication 6, dans lequel la phase solide portant des groupes amine primaire est sous la forme de particules libres ou sous la forme d'un dispositif de chromatographie.

8. Procédé selon la revendication 6 ou 7, dans lequel les particules en phase solide sont présentes en une proportion volumétrique située dans la plage allant de 1 % à 10 %, ou de 2,5 % à 7,5 %, ou de 4,5 % à 5,0 % par rapport au volume de récolte de culture cellulaire dans un milieu aqueux.

9. Procédé selon la revendication 6 ou 7, dans lequel la phase solide portant des groupes amine primaire est sous une forme choisie dans le groupe constitué par les colonnes garnies de particules poreuses, les colonnes garnies de nanofibres, les colonnes garnies de matériaux monolithiques, les hydrogels, les filtres à membrane de filtration poreuse, et les filtres pour filtration profonde.

10. Procédé selon l'une quelconque des revendications 6 à 9, dans lequel la surface amine primaire de la phase solide s'accompagne de résidus chimiques de caractère alternatif, par exemple d'amines secondaires, d'amines tertiaires, d'amines quaternaires, de résidus hydrophobes, de résidus de liaison hydrogène, de résidus d'affinité à un métal, ou de combinaisons de ceux-ci.

11. Procédé selon l'une quelconque des revendications 1 à 10, lequel procédé est effectué en présence d'un ou plusieurs sels, sucres, tensioactifs, ions métalliques divalents, allantoïne, polyéthylèneglycols, agents floculants, ou combinaisons de ceux-ci.

12. Procédé selon l'une quelconque des revendications 1 à 11, comprenant en outre les étapes de :
- réduction de la force ionique d'une solution comprenant le produit biologique souhaité,
- élimination d'autres contaminants par mise en contact de la solution avec un échangeur de cations, lequel échangeur de cations se lie aux contaminants,
- encore réduction de la force ionique de la solution comprenant le produit biologique souhaité,
- purification du produit biologique souhaité par mise en contact de la solution avec un échangeur de cations, lequel échangeur de cations se lie au produit biologique souhaité.
